# FASCICULE DE BREVET EUROPEEN

(11) **EP 2 866 853 B1**
(45) Date de publication et mention de la délivrance du brevet: **30.11.2016**
(21) Numéro de dépôt: 13733390.2
(22) Date de dépôt: 12.06.2013
(51) Int. Cl.: A61M 1/16, B65D 81/32

(54) **DISPOSITIF POUR LA DETECTION DE LA RUPTURE DE LA OU DES PAROIS DE SEPARATION DES COMPARTIMENTS D'UNE POCHE**
VORRICHTUNG ZUR ERKENNUNG VON BRÜCHEN IN WÄNDEN ZUR TRENNUNG DER FÄCHER EINES BEUTELS
DEVICE FOR DETECTING THE BREAKING OF THE WALL OR WALLS SEPARATING THE COMPARTMENTS OF A BAG

(30) Priorité: 28.06.2012 FR 1256172
(43) Date de publication de la demande: 06.05.2015
(73) Titulaire: Physidia, 49124 Saint-Barthélémy-d'Anjou (FR)
(72) Inventeur: HOUDOU, Michel, F-49000 Angers (FR); FERME, Philippe, F-49124 Le Plessis Grammoire (FR); MARINE, Julien, F-49000 Angers (FR)
(74) Mandataire: Dutreix, Hugues Ours
(86) Numéro de dépôt international: PCT/FR2013/051372
(87) Numéro de publication internationale: WO 2014/001680

(56) Documents cités:
- WO-A1-90/14293
- WO-A1-2011/073274
- WO-A2-2007/144427
- US-A1- 2002 066 678
- US-A1- 2003 118 779

## Description

La présente invention concerne de manière générale la détection de la rupture des parois de séparation des compartiments de poches.

L'invention concerne plus particulièrement un dispositif pour la détection de la rupture de la ou des parois de séparation des compartiments d'une poche, de préférence de dialysat, ledit dispositif comprenant un premier support, dit étage inférieur, sur lequel repose une poche de dialysat, et un deuxième support, dit étage supérieur, superposé audit étage inférieur.

Le dialysat est un liquide bien connu de l'homme du métier dont la composition est proche du sérum physiologique. Le dialysat comprend de l'eau d'une qualité suffisante sur le plan minéral et sur le plan bactériologique. Cette eau purifiée est additionnée à un concentré, sous forme de poudre de sels minéraux ou sous forme de concentré liquide, pour fabriquer le dialysat requis.

Dans le cadre de la dialyse d'un patient à l'aide d'une machine de dialyse, le dialysat utilisé est généralement un dialysat prêt à l'emploi contenu dans des poches. Les poches de dialysat sont connectées à la machine de dialyse, soit les unes après les autres, soit en parallèle au moyen d'une tuyauterie de type pieuvre.

Le document US 2002/066678 divulgue un système de poche avec plusieurs compartiments,

En particulier, il est connu d'utiliser un dialysat prêt à l'emploi, appelé tampon au lactate, qui se présente sous la forme d'une poche mono-compartiment. Cependant, les médecins préfèrent généralement utiliser un autre dialysat prêt à l'emploi, appelé tampon au bicarbonate, dont le contenu est plus facilement assimilable par le patient que le tampon au lactate.

Ledit tampon au bicarbonate se présente sous la forme d'une poche, qui comprend un compartiment contenant du bicarbonate et un autre compartiment contenant un liquide avec des ions susceptibles de précipiter avec le bicarbonate, tels que des ions calcium, à l'état mélangé des deux contenus. Les deux compartiments sont séparés l'un de l'autre par une paroi de séparation étanche qu'il convient de casser pour débuter l'opération de dialyse.

Les ions du liquide et le bicarbonate sont isolés dans deux compartiments de la poche pour éviter un risque de précipitation, c'est-à-dire de formation des sels. En outre, si les ions et le bicarbonate étaient préalablement mélangés dans la poche on observerait au bout d'un certain temps une altération du mélange.

Une fois la paroi de séparation de la poche cassée, les liquides se mélangent et le dialysat obtenu doit être utilisé dans les 48 ou 72 heures.

Il arrive cependant qu'une opération de dialyse soit démarrée alors que la paroi de séparation d'une poche ou de plusieurs poches n'est pas cassée, de sorte que le mélange entre les deux compartiments ne se fait pas.

Dans ce cas le liquide qui sort de la ou des poches ne présente pas les propriétés souhaitées pour une bonne dialyse du patient et risque de se révéler toxique pour le patient. Par exemple, la concentration en calcium risque de ne pas être adaptée à l'opération de dialyse.

Il est donc important de ne pas dialyser un patient à l'aide d'une poche de dialysat qui présente une paroi de séparation encore intacte entre deux compartiments. Il est cependant difficile et contraignant de s'assurer, pour l'opérateur ou le patient, que chaque paroi de séparation de chaque poche de dialysat est bien cassée, en particulier lorsqu'il s'agit d'un traitement en dehors d'une structure médicale, par exemple en hémodialyse quotidienne à domicile.

La présente invention a pour but de proposer un nouveau dispositif permettant de détecter aisément, et de manière fiable et rapide, l'état cassé ou non d'une paroi de séparation de compartiments d'une poche de dialysat.

A cet effet, l'invention a pour objet un dispositif pour la détection de la rupture de la ou des parois de séparation des compartiments d'une poche, de préférence une poche de dialysat, lesdits compartiments de la poche contenant différents contenus destinés à se mélanger après rupture de la ou des parois de séparation, ledit dispositif comprenant :
- un premier support, dit étage inférieur, sur lequel repose ladite poche,
- un deuxième support, dit étage supérieur, superposé audit étage inférieur,
caractérisé en ce que
lesdits étages inférieur et supérieur et la poche logée dans l'étage inférieur sont agencés de telle sorte que ledit étage supérieur comprend au moins une partie, dite partie de détection, qui, à l'état rompu de la ou des parois de séparation de la poche logée dans l'étage inférieur, est apte à venir dans une position rapprochée de l'étage inférieur, et qui, à l'état non rompu de la ou d'au moins une des parois de séparation de la poche logée dans l'étage inférieur, est apte à rester dans une position écartée dudit étage inférieur,
et en ce que le dispositif comprend des moyens de détection de la position relative de l'étage supérieur par rapport audit étage inférieur

Le dispositif selon l'invention permet ainsi de détecter une absence de rupture de paroi de séparation sans utiliser des moyens intrusifs qui risqueraient de contaminer le dialysat et/ou le circuit de distribution de dialysat.

En effet, la possibilité de détecter l'absence de rupture d'une paroi de séparation de poche par détection de la position relative entre l'étage supérieur et l'étage inférieur qui loge ladite poche, en particulier par détection de l'absence de contact entre une partie de l'étage supérieur et l'étage inférieur comme détaillé ci-après, est une méthode de détection simple, fiable et rapide.

Avant rupture de la paroi de séparation, les compartiments de la poche forment des protubérances d'épaisseur ou hauteur plus importante que la distance qui sépare la paroi de fond de l'étage supérieur de la paroi de fond de l'étage inférieur de sorte que, en l'absence de rupture de la paroi de séparation de compartiments de la poche, l'étage supérieur reste au moins partiellement écarté de l'étage inférieur.

Lorsque la paroi de séparation est cassée, les deux compartiments communiquent et les liquides se mélangent dans la zone préalablement obturée par la paroi de séparation, de sorte que le contenu de ces compartiments se répand dans toute la poche, ce qui réduit l'épaisseur de la poche et permet à l'étage supérieur de venir en position rapprochée, et en particulier à contact d'appui avec l'étage inférieur.

Selon une caractéristique avantageuse de l'invention, ladite position rapprochée de ladite partie de détection de l'étage supérieur par rapport audit étage inférieur correspond à une position de ladite partie de détection de l'étage supérieur en contact avec l'étage inférieur.

Autrement dit, lesdits moyens de détection sont configurés pour détecter le contact ou l'absence de contact entre ladite partie de détection de l'étage supérieur et l'étage inférieur, ce qui permet d'en déduire l'état cassé ou non d'une paroi de séparation de compartiment de poche.

En variante, on peut prévoir que les moyens de détection sont configurés pour déterminer la distance entre l'étage supérieur et l'étage inférieur afin de détecter l'état cassé ou non d'une paroi de séparation de compartiment de poche en fonction de la distance déterminée.

Selon une caractéristique avantageuse de l'invention, ledit dispositif comprend des moyens de signalisation configurés pour avertir l'opérateur lorsque ladite partie de détection de l'étage supérieur reste en position écartée de l'étage inférieur.

Selon une caractéristique avantageuse de l'invention, ladite poche étant destinée à alimenter en dialysat une machine de dialyse, ledit dispositif comprend des moyens de commande configurés pour empêcher le fonctionnement de la machine de dialyse lorsque ladite partie de détection de l'étage supérieur reste en position écartée de l'étage inférieur du dispositif.

Selon une caractéristique avantageuse de l'invention, ledit étage inférieur comprend une paroi de fond sur laquelle repose la poche et au moins deux parois opposées, appelées parois latérales, qui s'étendent sensiblement perpendiculairement à la paroi de fond, et ladite poche et lesdits étages sont configurés de telle sorte que, à l'état cassé de la ou des parois de séparation de la poche logée dans l'étage inférieur, ladite partie de détection de l'étage supérieur touche une paroi latérale de l'étage inférieur.

Selon une caractéristique avantageuse de l'invention, lesdits étages sont inclinés par rapport à l'horizontale.

Selon une caractéristique avantageuse de l'invention, lesdits moyens de détection comprennent une unité de traitement d'information configurée pour analyser une information représentative de l'état de contact de l'étage supérieur avec l'étage inférieur.

Selon une caractéristique avantageuse de l'invention, la ou chaque partie de détection de l'étage supérieur et/ou la ou chaque partie correspondante de l'étage inférieur est équipée d'un contacteur dont le contact ou l'absence de contact avec l'étage inférieur ou supérieur est apte à générer une information que l'unité de traitement est configurée pour analyser afin de détecter l'état de contact dudit contacteur.

Selon une caractéristique avantageuse de l'invention, la ou chaque partie de détection de l'étage supérieur est équipée d'un contacteur apte à venir en contact avec un contacteur ménagé sur une partie correspondante de l'étage inférieur à l'état cassé de la ou des parois de séparation de la poche logée dans l'étage inférieur, le contact ou l'absence de contact desdits contacteurs entre eux étant apte à générer une information que l'unité de traitement est configurée pour analyser afin de détecter l'état de contact desdits contacteurs.

Selon une caractéristique avantageuse de l'invention, le ou chaque contacteur est un capteur d'effort apte à émettre un signal à destination de l'unité de traitement lorsqu'un effort de valeur supérieure à une valeur seuil prédéfini lui est appliqué.

Selon une caractéristique avantageuse de l'invention, le ou chaque contacteur est un élément conducteur électrique qui forme un interrupteur électrique d'un circuit électrique auquel l'unité de traitement est raccordée pour déterminer l'état fermé ou ouvert dudit circuit.

L'invention sera bien comprise à la lecture de la description suivante d'exemples de réalisation, en référence aux dessins annexés dans lesquels :
- la figure 1 est une vue schématique montrant une poche présentant deux compartiments et une paroi de séparation non cassée;
- la figure 1A est une vue schématique de la poche de la figure 1 à l'état cassé de la paroi de séparation de ladite poche;
- la figure 2 est une vue schématique du dispositif selon l'invention, dont les différents étages logent des poches dont les parois de séparation des compartiments sont cassées;
- la figure 2A est une vue du dispositif de la figure 2 à l'état non cassé de la paroi de séparation des compartiments de l'une des poches ;

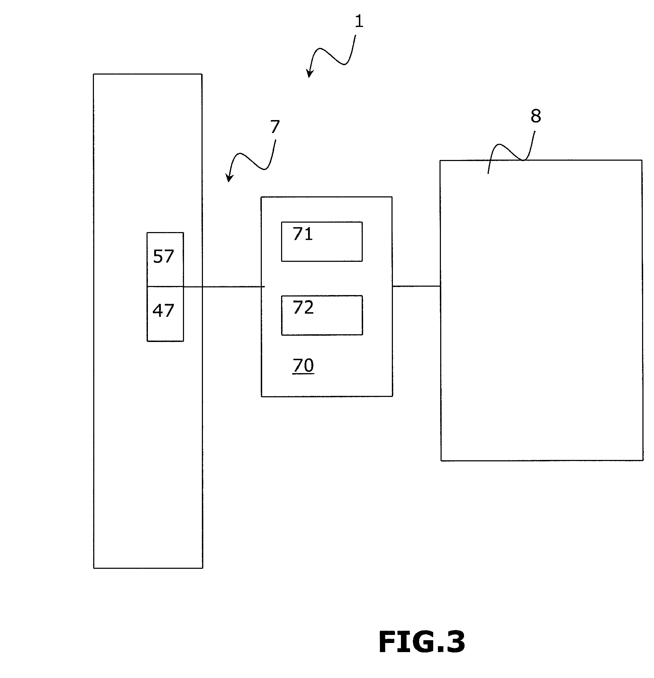

- la figure 3 est une vue schématique du dispositif selon l'invention associé à une machine de dialyse.

En référence aux figures et comme rappelé ci-dessus, l'invention concerne un dispositif 1 pour la détection de la rupture de la ou des parois de séparation 3 des compartiments 21, 22 d'une poche 2 de dialysat.

Chaque poche peut présenter deux compartiments ou plus. Généralement, les poches de dialysat présentent deux ou trois compartiments.

On entend par poche de dialysat une poche dont les compartiments présentent différents produits qui, à l'état mélangé, forme un dialysat utilisable pour alimenter le circuit de dialysat d'une machine de dialyse.

A titre d'exemple, l'un des compartiments peut contenir du bicarbonate et l'autre compartiment une solution ionique, par exemple des ions calcium.

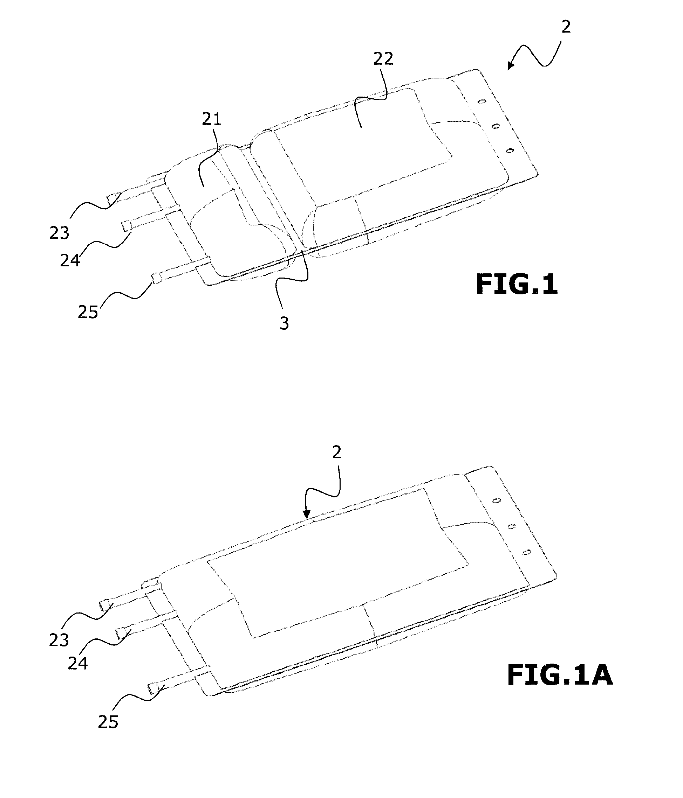

Chaque poche présente une sortie. Ladite sortie peut comprendre un ou plusieurs orifices de sortie 23, 24. Dans l'exemple illustré à la figure 1, l'orifice 25 de la poche 2 sert à remplir le petit compartiment 21 de la poche 2.

Dans la suite de la description, on décrit un mode de réalisation de l'invention appliqué à une poche 2 qui présente deux compartiments 21, 22 séparés par une paroi de séparation 3. Bien entendu, la description s'applique aussi à une poche comprenant une pluralité de parois de séparation. De même, on décrit par la suite la détection de l'absence de rupture d'une paroi de séparation de compartiment pour une poche logée dans un étage, mais l'invention s'applique aussi à la détection de l'absence de rupture d'une paroi de séparation de compartiment pour plusieurs poches logées dans différents étages.

Ledit dispositif comprend une structure support de poches qui présente un premier support 4, dit étage inférieur, sur lequel repose une poche 2 de dialysat, et un deuxième support 5, dit étage supérieur, superposé audit premier étage. La structure support peut être formée de plusieurs dispositifs superposés qui comprennent un étage en commun. En particulier, l'étage supérieur d'un premier dispositif joue le rôle d'étage inférieur d'un deuxième dispositif situé au dessus du premier dispositif. Ainsi, dans l'exemple illustré aux figures, 2 et 2A, l'étage supérieur 5 loge une autre poche 2 de dialysat.

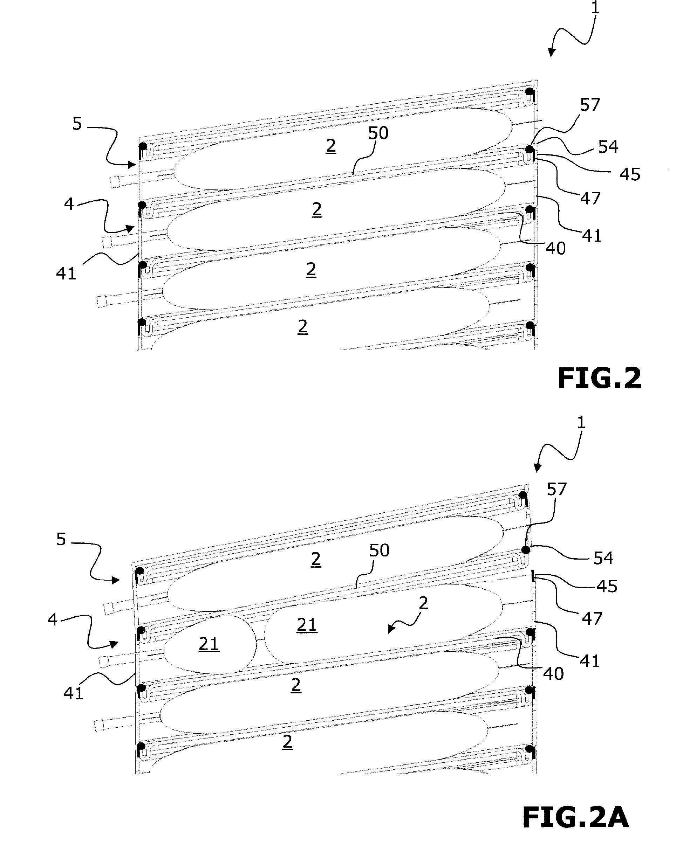

Dans l'exemple illustré aux figures, la structure support comprend aussi d'autres étages superposés les uns aux autres qui sont similaires ou identiques aux étages 4 et 5 détaillés ci-après. Le dernier étage peut être surmonté d'un simple couvercle comme illustré à la figure 2. Ladite structure support de poches forme ainsi un chargeur de poches à étages.

L'étage inférieur 4 comprend une paroi de fond 40 sur laquelle repose la poche 2 et au moins deux parois latérales opposées qui s'étendent sensiblement perpendiculairement à la paroi de fond, entre la partie avant et la partie arrière de l'étage. La partie avant d'un étage est celle au niveau de laquelle est située la sortie 23, 24 de la poche 2. Ledit étage inférieur 4 comprend aussi deux autres parois latérales 41 qui forment les parois avant et arrière dudit étage. Préférentiellement, l'étage supérieur 5 est similaire à l'étage inférieur 4 et comprend en particulier une paroi de fond 50 apte, comme détaillé ci-après, à venir par sa partie 54 arrière en appui sur le haut 45 de la partie latérale 41 arrière de l'étage inférieur 4 à l'état cassé de la paroi de séparation 3 de la poche 2 logée dans l'étage inférieur 4.

Lesdits étages 4, 5 sont inclinés par rapport à l'horizontale de sorte que la sortie de chaque poche 2 se situe en partie basse de l'étage qui loge ladite poche. Lesdits étages s'étendent sensiblement parallèlement les uns aux autres à l'état cassé des parois de séparation de compartiments des poches.

Comme illustré à la figure 2A, à l'état non rompu de la paroi de séparation de la poche 2 logée dans l'étage 4 inférieur, les compartiments 21, 22 de la poche 2 forment des boursouflures dont l'épaisseur ou hauteur empêche l'étage supérieur 5 de venir en appui au niveau de sa partie arrière 54 contre la partie correspondante 45 de l'étage inférieur 4 qui loge ladite poche 2.

A l'inverse, comme illustré à la figure 2, lorsque la paroi de séparation de la poche est correctement cassée, l'épaisseur des compartiments diminue puisque leur contenu se répand dans la zone de séparation desdits compartiments précédemment obturée par la paroi de séparation. Il en résulte que la poche n'empêche plus l'étage 5 supérieur de venir complètement en appui sur l'étage 4 inférieur, en particulier au niveau de sa partie 54.

Ainsi, les étages inférieur 4 et supérieur 5 et la poche 2 logée dans l'étage inférieur 4 sont dimensionnés de telle sorte que ledit étage supérieur 5 présente au moins une partie 54, dite partie de détection ou encore partie de contact, qui, à l'état rompu de la paroi de séparation 3 de la poche logée dans l'étage inférieur, vient en contact de l'étage inférieur, et qui, à l'état non rompu de la paroi de séparation de la poche logée dans l'étage inférieur, reste écartée dudit étage inférieur.

Normalement, la rupture de la paroi de séparation 3 des compartiments d'une poche 2 logée dans un étage est obtenue par application d'un effort de pression sur ladite poche. On peut également prévoir que la rupture de la paroi de séparation 3 des compartiments d'une poche 2 logée dans un étage inférieur s'opère sous le poids du ou des étage(s) supérieur(s) au(x)quel(s) un effort de pression supplémentaire peut être appliqué.

Il est important de pouvoir détecter une absence de rupture de paroi de séparation de compartiments de poche pour ne pas démarrer une opération de dialyse avec un dialysat de mauvaise qualité.

A cet effet, le dispositif comprend des moyens de détection de contact 7 configurés pour détecter le contact ou l'absence de contact entre ladite partie de contact 54 de l'étage supérieur 5 et ledit étage inférieur 4.

Lesdits moyens de détection de contact 7 comprennent des contacteurs 57, 47 positionnés sur les étages inférieur 4 et supérieur 5. En particulier, des contacteurs sont positionnés au niveau de ladite partie 54 de contact de l'étage supérieur et de la partie 45 correspondante de l'étage inférieur 4.

Les moyens de détection de contact 7 comprennent également une unité de traitement 70 qui permet de détecter le contact ou l'absence de contact des, ou d'une partie des, contacteurs qui équipent les étages avec les contacteurs correspondants des autres étages. On peut en particulier prévoir que ladite unité de traitement 70 soit apte à recevoir et analyser un signal résultant de la fermeture ou de l'ouverture du contact entre les contacteurs de deux étages. Ladite unité de traitement 70 est formée par un système électronique et/ou informatique qui comprend par exemple un microprocesseur.

On entend par contacteur un organe apte à venir en contact avec un autre objet, tel qu'un autre contacteur, et dont l'état de contact se traduit par un signal ou une absence de signal apte à être analysé(e) par l'unité de traitement 70.

Les contacteurs peuvent être réalisés de différentes manières. Selon un mode de réalisation particulier, le ou chaque contacteur peut être un élément conducteur électrique qui forme, éventuellement en coopération avec un ou d'autres contacteurs, un interrupteur électrique d'un circuit électrique auquel l'unité de traitement 70 est raccordée pour pouvoir déterminer l'état fermé ou ouvert dudit circuit électrique. On peut ainsi prévoir que le contacteur 57 de l'étage 5 supérieur et celui 47 de l'étage 4 inférieur forment deux bornes d'un circuit électrique qui est fermé à l'état appuyé du contacteur 57 sur le contacteur 47, et qui à l'inverse est ouvert à l'état écarté l'un de l'autre desdits contacteurs.

En variante, le ou chaque contacteur peut être un capteur d'effort apte à émettre un signal à destination de l'unité de traitement 70 lorsqu'il est soumis à un effort de valeur supérieure à une valeur seuil prédéfinie. Ledit effort résulte du contact entre l'étage supérieur et l'étage inférieur. Ainsi, on peut prévoir qu'une absence de réception de signal par l'unité de traitement 70 soit interprétée par ladite unité comme une absence de contact entre les étages correspondant du dispositif associés à ce contacteur.

Dans l'exemple illustré à la figure 2A pour lequel la paroi de séparation 3 de la poche 2 de l'étage inférieur 4 n'est pas cassée, le contact est ouvert entre le contacteur 57 qui équipe la partie arrière 54 de l'étage supérieur 5 et le contacteur 47 qui équipe la partie correspondante 45 de l'étage inférieur 4. A l'inverse, dans l'exemple illustré à la figure 2 pour lequel la paroi de séparation 3 de la poche 2 de l'étage inférieur 4 est bien cassée, le contact est fermé entre le contacteur 57 qui équipe la partie arrière 54 de l'étage supérieur 5 et le contacteur 47 qui équipe la partie correspondante 45 de l'étage inférieur 4.

Les contacteurs peuvent être positionnés sur les différentes parties d'un étage qui sont susceptibles de rester écartées d'un étage voisin en cas de non rupture de paroi de détection. Ainsi, on peut prévoir de disposer des contacteurs au niveau de chacun des coins de la partie arrière de chaque étage et éventuellement comme illustré aux figures 2 et 2A au niveau de la partie avant de chaque étage.

Avantageusement, ladite unité de traitement 70 comprend des moyens de signalisation 71 configurés pour avertir l'opérateur lorsqu'une absence de contact entre ladite partie de contact 54 de l'étage supérieur 5 et l'étage inférieur 4 est détectée. On peut aussi prévoir que ladite unité de traitement 70 soit munie de moyens de pilotage 72 configurés pour empêcher le fonctionnement de la machine de dialyse 8 en l'absence de contact entre ladite partie de contact 54 de l'étage supérieur 5 et l'étage inférieur 4.

Lesdits moyens de signalisation 71 et lesdits moyens de pilotage 72 inclus dans l'unité de traitement 70 peuvent être réalisés sous forme d'instructions informatiques.

On peut aussi prévoir que ledit dispositif comprend des moyens de transmission à la machine de dialyse 8 d'une information indiquant l'état des contacteurs (par une liaison sans fil ou filaire).

La présente invention n'est nullement limitée aux modes de réalisation décrits et représentés, mais l'homme du métier saura y apporter toute variante conforme à son esprit.

## Revendications

1. Dispositif (1) pour la détection de la rupture de la ou des parois de séparation (3) des compartiments (21, 22) d'une poche (2), de préférence une poche de dialysat, lesdits compartiments (21, 22) de la poche contenant différents contenus destinés à se mélanger après rupture de la ou des parois de séparation (3), ledit dispositif comprenant :
- un premier support (4), dit étage inférieur, sur lequel repose ladite poche (2),
- un deuxième support (5), dit étage supérieur, superposé audit étage inférieur,
**caractérisé en ce que**
lesdits étages inférieur (4) et supérieur (5) et la poche logée dans l'étage inférieur (4) sont agencés de telle sorte que ledit étage supérieur (5) comprend au moins une partie (54), dite partie de détection, qui, à l'état rompu de la ou des parois de séparation (3) de la poche (2) logée dans l'étage inférieur (4), est apte à venir dans une position rapprochée de l'étage inférieur (4), et qui, à l'état non rompu de la ou d'au moins une des parois de séparation (3) de la poche (2) logée dans l'étage inférieur (4), est apte à rester dans une position écartée dudit étage inférieur (4),
et **en ce que** le dispositif comprend des moyens de détection (7) de la position relative de l'étage supérieur (5) par rapport audit étage inférieur (4).

2. Dispositif selon la revendication 1, **caractérisé en ce que** ladite position rapprochée de ladite partie de détection (54) de l'étage supérieur (5) par rapport audit étage inférieur (4) est une position de ladite partie de détection (54) de l'étage supérieur (5) en contact avec l'étage inférieur (4).

3. Dispositif selon l'une des revendications 1 ou 2, **caractérisé en ce que** ledit dispositif (1) comprend des moyens de signalisation (71) configurés pour avertir l'opérateur lorsque ladite partie de détection (54) de l'étage supérieur (5) reste en position écartée de l'étage inférieur (4).

4. Dispositif selon l'une des revendications 1 à 3, **caractérisé en ce que**, ladite poche (2) étant destinée à alimenter en dialysat une machine de dialyse (8), ledit dispositif comprend des moyens de commande (72) configurés pour empêcher le fonctionnement de la machine de dialyse (8) lorsque ladite partie de détection (54) de l'étage supérieur (5) reste en position écartée de l'étage inférieur (4) du dispositif.

5. Dispositif selon l'une des revendications précédentes prise en combinaison de la revendication 2, **caractérisé en ce que** ledit étage inférieur (4) comprend une paroi de fond (40) sur laquelle repose la poche (2) et au moins deux parois (41) opposées, appelées parois latérales, qui s'étendent sensiblement perpendiculairement à la paroi de fond,
et **en ce que** ladite poche (2) et lesdits étages (4, 5) sont configurés de telle sorte que, à l'état cassé de la ou des parois de séparation (3) de la poche (2) logée dans l'étage inférieur (4), ladite partie de détection (54) de l'étage supérieur (5) touche une paroi latérale de l'étage inférieur (4).

6. Dispositif selon l'une des revendications précédentes, **caractérisé en ce que** lesdits étages (4, 5) sont inclinés par rapport à l'horizontale.

7. Dispositif selon l'une des revendications précédentes prise en combinaison de la revendication 2, **caractérisé en ce que** lesdits moyens de détection (7) comprennent une unité de traitement (70) d'information configurée pour analyser une information représentative de l'état de contact de l'étage supérieur (5) avec l'étage inférieur (4).

8. Dispositif selon la revendication 7, **caractérisé en ce que** la ou chaque partie de détection (54) de l'étage supérieur (5) et/ou la ou chaque partie correspondante (45) de l'étage inférieur (4) est équipée d'un contacteur (57, 47) dont le contact ou l'absence de contact avec l'étage inférieur (4) ou supérieur (5) est apte à générer une information que l'unité de traitement (70) est configurée pour analyser afin de détecter l'état de contact dudit contacteur (57, 47).

9. Dispositif selon la revendication 7, **caractérisé en ce que** la ou chaque partie de détection (54) de l'étage supérieur (5) est équipée d'un contacteur (57) apte à venir en contact avec un contacteur (47) ménagé sur une partie correspondante de l'étage inférieur (4) à l'état cassé de la ou des parois de séparation (3) de la poche (2) logée dans l'étage inférieur (4),
le contact ou l'absence de contact desdits contacteurs (57, 47) étant apte à générer une information que l'unité de traitement (70) est configurée pour analyser afin de détecter l'état de contact desdits contacteurs (57, 47).

10. Dispositif selon l'une des revendications 8 ou 9, **caractérisé en ce que** le ou chaque contacteur (57, 47) est un capteur d'effort apte à émettre un signal à destination de l'unité de traitement (70) lorsqu'il subit un effort de valeur supérieure à une valeur seuil prédéfinie.

11. Dispositif selon l'une des revendications 8 ou 9, **caractérisé en ce que** le ou chaque contacteur (57, 47) est un élément conducteur électrique qui forme un interrupteur électrique d'un circuit électrique auquel l'unité de traitement (70) est raccordé pour déterminer l'état fermé ou ouvert dudit circuit.

## Patentansprüche

1. Vorrichtung (1) zum Erkennen von Brüchen der Trennwand / Trennwände (3) der Fächer (21, 22) eines Beutels (2), vorzugsweise eines Dialysatbeutels, wobei die Fächer (21, 22) des Beutels verschiedene Inhalte enthalten, die bestimmt sind, sich nach Bruch der Trennwand / Trennwände (3) zu vermischen, wobei die Vorrichtung umfasst:
- eine als untere Etage bezeichnete erste Unterlage (4), auf welcher der Beutel (2) ruht,
- eine als obere Etage bezeichnete zweite Unterlage (5) über der unteren Etage, **dadurch gekennzeichnet, dass**
die untere (4) und obere (5) Etage und der in der unteren Etage (4) untergebrachte Beutel derart ausgebildet sind, dass die obere Etage (5) mindestens einen als Erkennungsteil bezeichneten Teil (54) umfasst, der im zerbrochenen Zustand der Trennwand / Trennwände (3) des in der unteren Etage (4) untergebrachten Beutels (2) imstande ist, in eine an die untere Etage (4) angenäherte Position zu kommen und der im nicht zerbrochenen Zustand der oder mindestens einer der Trennwände (3) des in der unteren Etage (4) untergebrachten Beutels (2) imstande ist, in einer von der unteren Etage (4) beabstandeten Position zu verbleiben,
und dass die Vorrichtung Erkennungsmittel (7) der relativen Position der oberen Etage (5) in Bezug zur unteren Etage (4) umfasst.

2. Vorrichtung nach Anspruch 1, **dadurch gekennzeichnet, dass** die angenäherte Position des Erkennungsteils (54) der oberen Etage (5) in Bezug zur unteren Etage (4) eine Position des Erkennungsteils (54) der oberen Etage (5) im Kontakt mit der unteren Etage (4) ist.

3. Vorrichtung nach einem der Ansprüche 1 oder 2, **dadurch gekennzeichnet, dass** die Vorrichtung (1) Anzeigemittel (71) umfasst, die konfiguriert sind, um den Bediener zu warnen, wenn der Erkennungsteil (54) der oberen Etage (5) in von der unteren Etage (4) beabstandeten Position verbleibt.

4. Vorrichtung nach einem der Ansprüche 1 bis 3, **dadurch gekennzeichnet, dass**, wobei der Beutel (2) bestimmt ist, eine Dialysemaschine (8) mit Dialysat zu versorgen, die Vorrichtung Steuermittel (72) umfasst, die konfiguriert sind, um den Betrieb der Dialysemaschine (8) zu verhindern, wenn der Erkennungsteil (54) der oberen Etage (5) in von der unteren Etage (4) der Vorrichtung beabstandeten Position verbleibt.

5. Vorrichtung nach einem der vorangehenden Ansprüche, herangezogen in Kombination mit Anspruch 2, **dadurch gekennzeichnet, dass** die untere Etage (4) eine Bodenwand (40) umfasst, auf welcher der Beutel (2) ruht, und mindestens zwei als Seitenwände bezeichnete gegenüberliegende Wände (41), die sich etwa senkrecht zur Bodenwand erstrecken,
und dass der Beutel (2) und die Etagen (4, 5) derart konfiguriert sind, dass in zerbrochenem Zustand der Trennwand / Trennwände (3) des in der unteren Etage (4) untergebrachten Beutels (2) der Erkennungsteil (54) der oberen Etage (5) eine Seitenwand der unteren Etage (4) berührt.

6. Vorrichtung nach einem der vorangehenden Ansprüche, **dadurch gekennzeichnet, dass** die Etagen (4, 5) in Bezug auf die Horizontale geneigt sind.

7. Vorrichtung nach einem der vorangehenden Ansprüche, herangezogen in Kombination mit Anspruch 2, **dadurch gekennzeichnet, dass** die Erkennungsmittel (7) eine Informationsverarbeitungseinheit (70) umfassen, die konfiguriert ist, um eine repräsentative Information des Kontaktzustands der oberen Etage (5) mit der unteren Etage (4) zu analysieren.

8. Vorrichtung nach Anspruch 7, **dadurch gekennzeichnet, dass** der oder jeder Erkennungsteil (54) der oberen Etage (5) und/oder der oder jeder entsprechende Teil (45) der unteren Etage (4) mit einem Schalter (57, 47) ausgestattet ist, dessen Kontakt oder fehlender Kontakt mit der unteren (4) oder oberen (5) Etage imstande ist, eine Information zu erzeugen, dass die Verarbeitungseinheit (70) konfiguriert ist, um zu analysieren, um den Kontaktzustand des Schalters (57, 47) zu erkennen.

9. Vorrichtung nach Anspruch 7, **dadurch gekennzeichnet, dass** der oder jeder Erkennungsteil (54) der oberen Etage (5) mit einem Schalter (57) ausgestattet ist, der imstande ist, mit einem Schalter (47) in Kontakt zu kommen, der auf einem entsprechenden Teil der unteren Etage (4) im zerbrochenen Zustand der Trennwand / Trennwände (3) des in der unteren Etage (4) untergebrachten Beutels (2) ausgebildet ist,
wobei der Kontakt oder der fehlende Kontakt der Schalter (57, 47) imstande ist, eine Information zu erzeugen, dass die Verarbeitungseinheit (70) konfiguriert ist, um zu analysieren, um den Kontaktzustand der Schalter (57, 47) zu erkennen.

10. Vorrichtung nach einem der Ansprüche 8 oder 9, **dadurch gekennzeichnet, dass** der oder jeder Schalter (57, 47) ein Kraftsensor ist, der imstande ist, ein Signal an die Verarbeitungseinheit (70) zu senden, wenn auf ihn eine Kraft wirkt, deren Wert über einem vorbestimmten Grenzwert liegt.

11. Vorrichtung nach einem der Ansprüche 8 oder 9, **dadurch gekennzeichnet, dass** der oder jeder Schalter (57, 47) ein elektrisch leitendes Element ist, der einen elektrischen Unterbrecher eines Stromkreises bildet, an den die Verarbeitungseinheit (70) angeschlossen ist, um den geschlossenen oder geöffneten Zustand des Kreises zu bestimmen.

## Claims

1. A device (1) for detecting the breaking of the wall(s) (3) separating the compartments (21, 22) of a bag (2), preferably a dialysate bag, said compartments (21, 22) of the bag containing different contents intended to mix after breaking of the separating wall(s) (3), said device comprising:
- a first support (4), called lower stage, on which said bag (2) rests,
- a second support (5), called upper stage, superimposed on said lower stage,
**characterized in that**
said lower (4) and upper (5) stages and the bag housed in the lower stage (4) are arranged such that said upper stage (5) comprises at least one part (54), called detection part, which, in a broken state of the separating wall(s) (3) of the bag (2) housed in the lower stage (4), is able to come into a position close to the lower stage (4), and which, in the unbroken state of the or at least one of the separating walls (3) of the bag (2) housed in the lower stage (4), is able to remain in a position separated from said lower stage (4), and **in that** the device comprises means (7) for detecting the relative position of the upper stage (5) with respect to said lower stage (4).

2. The device according to claim 1, **characterized in that** said close position of said detection part (54) of the upper stage (5) relative to the lower stage (4) is a position of said detection part (54) of the upper stage (5) in contact with the lower stage (4).

3. The device according to one of claims 1 or 2, **characterized in that** said device (1) comprises signaling means (71) configured to notify the operator when said detection part (54) of the upper stage (5) remains in the separated position from the lower stage (4).

4. The device according to one of claims 1 to 3, **characterized in that**, said bag (2) being intended to supply dialysate to a dialysis machine (8), said device comprises control means (72) configured to prevent the operation of the dialysis machine (8) when said detection part (54) of the upper stage (5) remains in the separated position from the lower stage (4) of the device.

5. The device according to one of the preceding claims combined with claim 2, **characterized in that** said lower stage (4) comprises a bottom wall (40) on which the bag (2) rests and at least two opposite walls (41), called side walls, that extend substantially perpendicular to the bottom wall,
and **in that** said pouch (2) and said stages (4, 5) are configured such that, in the broken state of the separating wall(s) (3) of the pouch (2) housed in the lower stage (4), said detection part (54) of the upper stage (5) touches a side wall of the lower stage (4).

6. The device according to one of the preceding claims, **characterized in that** said stages (4, 5) are inclined relative to the horizontal.

7. The device according to one of the preceding claims combined with claim 2, **characterized in that** said detection means (7) comprises an information processing unit (70) configured to analyze a piece of information representative of the contact state of the upper stage (5) with the lower stage (4).

8. The device according to claim 7, **characterized in that** the or each detection part (54) of the upper stage (5) and/or the or each corresponding part (45) of the lower stage (4) is equipped with a contactor (57, 47) whereof the contact or absence of contact with the lower stage (4) or upper stage (5) is able to generate a piece of information that the processing unit (70) is configured to analyze in order to detect the contact state of said contactor (57, 47).

9. The device according to claim 7, **characterized in that** the or each detection part (54) of the upper stage (5) is equipped with a contactor (57) able to come into contact with a contactor (47) arranged on a corresponding part of the lower stage (4) in the broken state of the separating wall(s) (3) of the bag (2) housed in the lower stage (4),
the contact or absence of contact of said contactors (57, 47) being able to generate a piece of information that the processing unit (70) is configured to analyze in order to detect the contact state of said contactors (57, 47).

10. The device according to one of claims 8 or 9, **characterized in that** the or each contactor (57, 47) is a force sensor able to emit a signal intended for the processing unit (70) when it undergoes a force with a value exceeding a predefined threshold.

11. The device according to one of claims 8 or 9, **characterized in that** the or each contactor (57, 47) is an electrical conductor that forms an electrical switch of an electrical circuit to which the processing unit (70) is connected to determine the closed or open state of said circuit.
